# FASCICULE DE BREVET EUROPEEN

(11) **EP 0 648 515 B1**
(45) Date de publication et mention de la délivrance du brevet: **23.04.2003**
(21) Numéro de dépôt: 94440064.7
(22) Date de dépôt: 13.10.1994
(51) Int. Cl.: A61N 5/04

(54) **Antenne pour le chauffage de tissus par micro-ondes et sonde comportant une ou plusieurs de ces antennes**
Antenne zur Heizung von Geweben durch Mickrowellen und Sonde mit einer oder mehrerer Antennen
Antenna for microwave heating of tissue and catheter with one or more antennas

(30) Priorité: 15.10.1993 FR 9312472
(43) Date de publication de la demande: 19.04.1995
(73) Titulaire: BRUKER SA, 67160 Wissembourg (FR)
(72) Inventeur: Weiss, Michel, F-67240 Gries (FR); Ringeisen, Victor, F-67160 Riedseltz (FR)
(74) Mandataire: Nuss, Pierre

(56) Documents cités:
- WO-A-89/11311
- WO-A-92/07622
- WO-A-93/17756
- US-A- 4 825 880
- KRAUS JD: "Antennas", 1988, MCGRAW-HILL, NEW YORK

## Description

La présente invention concerne le domaine des techniques d'hyperthermie ou de thermothérapie, plus particulièrement les antennes, applicateurs ou sondes destinés à être introduits dans des cavités ou des conduits, notamment de faible section, d'un corps vivant, animal ou humain, et a pour objet une antenne pour le chauffage de tissus par micro-ondes et une sonde comprenant une ou plusieurs de ces antennes.

Il existe actuellement déjà différents types d'applicateurs ou de sondes pour le chauffage par micro-ondes de tissus vivants, notamment des applicateurs intra-urétraux destinés au traitement d'affections de la prostate et en particulier de l'hypertrophie bénigne de la prostate.

La plupart de ces applicateurs comporte des antennes filaires, obtenues en dénudant l'extrémité d'un câble coaxial de manière à exposer le conducteur filaire central. Des applicateurs de ce type sont notamment décrits dans le brevet français FR-A-2 421 628.

Toutefois ces applicateurs à antennes filaires réalisent un chauffage inhomogène du milieu environnant et focalisent notamment une part importante de la puissance émise dans un volume ou zone très restreint, aussi appelé point chaud. Ainsi, au cours d'un traitement par hyperthermie ou thermothérapie, il y a rapidement apparition d'une sensation de douleur pour le patient traité au niveau du point chaud de l'applicateur, ce qui limite l'élévation de température dans le zones adjacentes audit point chaud.

Il en résulte une irradiation insuffisante pour provoquer l'effet thérapeutique souhaité sur toute la zone de tissus à traiter, plus particulièrement lorsque cette dernière présente une forme allongée et est disposée autour de la cavité ou du conduit, dans lequel l'applicateur est inséré, sur une faible épaisseur.

En outre, le dépôt de puissance opéré par un tel applicateur est très irrégulier au niveau du volume entourant l'antenne rayonnante et on relève une dissymétrie du lobe de rayonnement avec un étirement vers l'arrière du dépôt de puissance, résultant en un chauffage néfaste de tissus sains, non destinés à être traités.

Par ailleurs, on connaît également des applicateurs comportant, en tant qu'élément rayonnant, un solénoïde ou plusieurs solénoïdes groupés, constituant une ou des boucles de courant fermées, alimentées en série ou séparément, permettant l'émission de champs magnétiques radio-fréquence. De tels applicateurs sont notamment connus par les documents EP-A-0 462 302 et WO 92/07 622.

Néanmoins, la répartition et le dépôt de puissance obtenus au moyen de ces derniers applicateurs n'est pas non plus homogène et symétrique et ils ne permettent pas non plus un chauffage efficace et uniforme d'un volume délimité de tissus, ce, notamment, pour des volumes de formes quelconques situés autour de la cavité, du canal ou du conduit recevant ledit applicateur. De plus, la mise en oeuvre de plusieurs bobines nécessite un dispositif d'alimentation compliqué et entraîne une structure volumineuse et complexe pour ledit applicateur.

Par le document WO 89/11311, on connaît notamment une antenne présentant sensiblement les caractéristiques mentionnées dans le préambule de la revendication 1.

Le problème posé à la présente invention consiste principalement à améliorer l'antenne précitée pour obtenir une antenne et une sonde comportant au moins une telle antenne présentant une structure simple et des dimensions réduites et permettant de réaliser un chauffage efficace et uniforme d'un volume donné situé dans ou autour d'une cavité ou autour d'un conduit, dans laquelle ou dans lequel ladite antenne ou ladite sonde a été introduite, sans provoquer pour autant un chauffage involontaire des tissus disposés en dehors dudit volume, même ceux adjacents à ce dernier.

Par conséquent, cette antenne et cette sonde devront permettre un dépôt de puissance et d'énergie rayonnées présentant la plus grande homogénéité possible pour un volume considéré s'étendant autour de ladite antenne ou de ladite sonde et ayant une forme donnée, mais également générer un lobe de rayonnement de forme variable par compositions de champs, tel qu'il aboutisse à une décroissance rapide dudit dépôt de puissance et d'énergie aux limites dudit volume considéré, afin d'éviter une irradiation involontaire des tissus et organes situés, de part et d'autre, en dehors dudit volume.

En outre, ladite antenne et ladite sonde devront également pouvoir être utilisées simultanément en tant que capteurs de bruit thermique radiofréquence, afin de déterminer par radiométrie micro-onde la température du volume chauffé, par un fonctionnement en réception en alternance avec le fonctionnement à l'émission pour le chauffage.

A cet effet, la présente invention a pour objet une antenne pour le chauffage de tissus par micro-ondes, destinée à être insérée dans une cavité ou un conduit, naturel ou réalisé pour les besoins d'un traitement, d'un corps vivant, notamment humain, qui est du type décrit dans le préambule de la revendication 1 et présente en combinaison les caractéristiques de la partie caractérisante de cette revendication 1.

L'invention a également pour objet une sonde pour le chauffage de tissus par micro-ondes et pour la mesure de température par radiométrie micro-onde, caractérisée en ce qu'elle comprend au moins une antenne telle que décrite ci-dessus.

L'invention sera mieux comprise grâce à la description ci-après, qui se rapporte à un mode de réalisation préféré, donné à titre d'exemple non limitatif, et expliqué avec référence aux dessins schématiques annexés, dans lesquels:
la figure 1 est une vue en élévation latérale et en coupe partielle d'une antenne conforme à l'invention,
la figure 2 représente une portion d'un diagramme de rayonnement montrant le dépôt d'énergie obtenu au moyen d'une antenne telle que représentée à la figure 1, dans le cas d'un traitement de la prostate par voie urétrale et comportant, en superpostion, différentes courbes à 3 dB du diagramme de rayonnement en réception radiométrique obtenues avec la même antenne;
la figure 3 représente la courbe d'adaptation de l'antenne représentée à la figure 1, en fonction de la fréquence, et,
la figure 4 est une vue en perspective d'une sonde conforme à l'invention, comprenant plusieurs antennes selon l'invention.

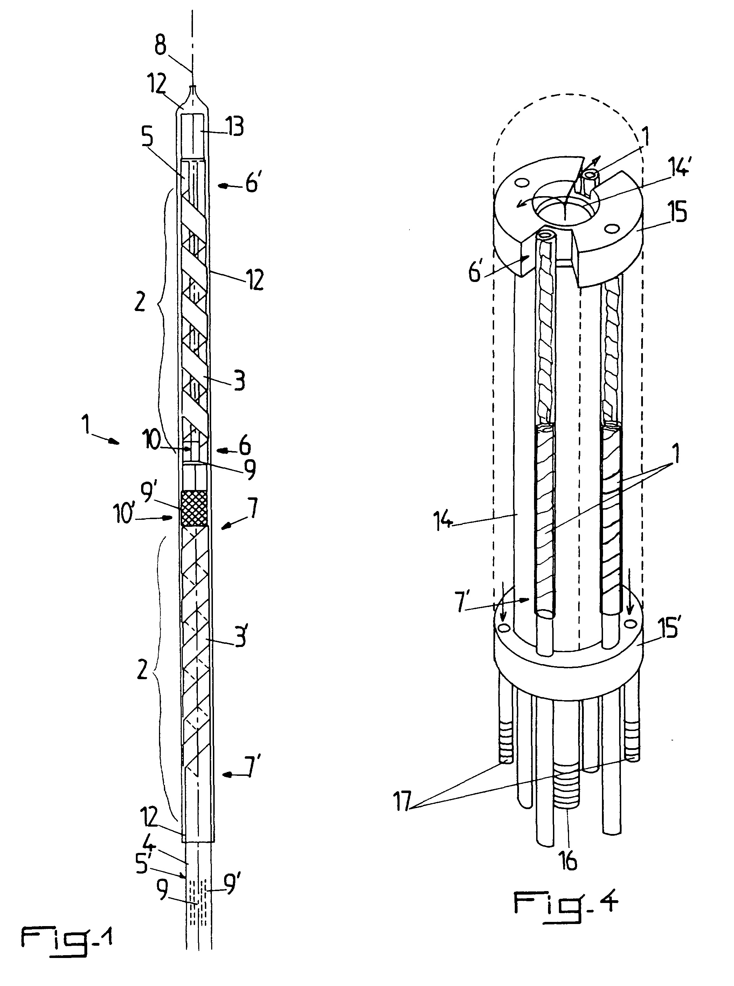

Conformément à l'invention et comme le montre la figure 1 des dessins annexés, l'antenne 1 est principalement constituée par deux lignes à retard 3, 3' rayonnantes, disposées au niveau de l'extrémité libre d'une ligne d'alimentation 4 et formées chacune par un ruban ou une bande de matériau conducteur, autorisant également, en alternance avec le chauffage des tissus, une réception des signaux de bruit thermique des tissus chauffés dans une bande de fréquence de 2 GHz à 4 GHz, en vue d'une détermination de la température par mesure radiométrique.

Ladite ligne d'alimentation 4 est reliée par son extrémité opposée à la partie rayonnante 2, à un dispositif de génération de micro-ondes, au moyen d'un connecteur adapté, et pourra être de type quelconque (bifilaire, coaxiale ou autre), mais présentera une longueur et une rigidité suffisantes, tout en étant flexible, pour permettre l'introduction et la mise en place interne de ladite antenne 1 ou de la sonde comportant celle-ci dans une cavité ou un conduit donné(e).

Le champ créé par une telle ligne à retard 3, 3' rayonnante sera donc de nature électrique, et non magnétique comme pour une bobine formant boucle de courant.

Conformément à un mode de réalisation préférentiel de l'invention et comme le montre la figure 1 des dessins annexés, l'antenne 1 comporte deux lignes à retard rayonnantes 3 et 3', se présentant sous la forme de deux portions de conducteur distinctes à structure hélicoïdale, montées sur des supports d'enroulement 5 et 5' en des matériaux diélectriques et dont les axes de symétrie longitudinaux 8 sont confondus.

Comme le montre également la figure 1 des dessins annexés, les deux portions de conducteur hélicoïdales peuvent être disposées de manière contiguë et connectées, au niveau de leurs extrémités adjacentes 6 et 7, aux deux conducteurs 9 et 9' de la ligne d'alimentation 4, leurs extrémités mutuellement opposées 6' et 7' étant non connectées, constituant ainsi un dipôle rayonnant alimenté en son centre.

Les caractéristiques de rayonnement et de réception de l'antenne 1 selon l'invention, telles que, par exemple, forme et dimension du lobe de rayonnement, peuvent être déterminées en réglant les paramètres structurels des deux lignes à retard rayonnantes 3 et 3'.

Ainsi, afin d'obtenir des effets compensatoires mutuels et d'aboutir notamment à des lobes de rayonnement symétriques, les deux portions de conducteur hélicoïdales 3 et 3' peuvent présenter des sens d'enroulement opposés en partant de leurs extrémités 6 et 7 adjacentes respectives, et, le cas échéant, des longueurs et des structures identiques permettant une composition de champs et de phases entre les deux lignes à retard rayonnantes et entre les segments élémentaires rayonnants de chaque ligne à retard.

De même, selon une caractéristique de l'invention représentée à la figure 1 des dessins annexés, chaque portion de conducteur hélicoïdale 3, 3' peut être avantageusement constituée par un ruban en un matériau conducteur dont la largeur, le pas d'enroulement, le diamètre d'enroulement et le nombre de spires formées sont déterminés en fonction des caractéristiques souhaitées, notamment de la forme du diagramme de rayonnement et de l'adaptation de l'antenne 1 à la fréquence désirée; certains de ces paramètres, tels que le diamètre d'enroulement, la largeur du ruban ou le pas d'enroulement, pouvant varier pour une même ligne à retard 3 ou 3' sur l'étendue longitudinale de cette dernière.

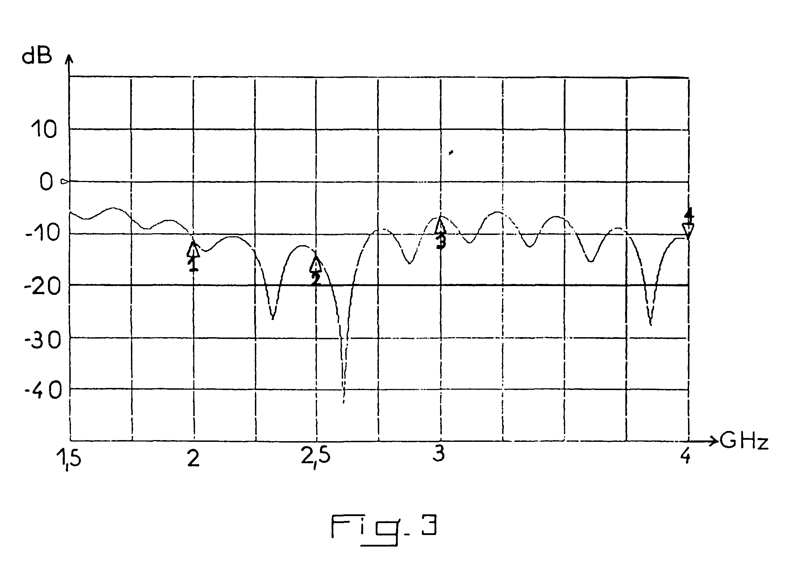

Enfin, en tenant compte du milieu dissipatif environnant que constitue les tissus à traiter, et en vue d'obtenir des performances de chauffage maximales pour un générateur micro-ondes de 915 MHz ou de 434 MHz, la largeur, le pas d'enroulement, le diamètre d'enroulement et le nombre de spires formées sont déterminés en fonction des caractéristiques, notamment de la forme du diagramme de rayonnement souhaité (Figure 3).

Selon un mode de réalisation préférée de l'invention, la ligne d'alimentation 4 consiste en une ligne de transmission à structure coaxiale (Figure 1).

Comme le montre également la figure 1 des dessins annexés, la longueur des portions de conducteur hélicoïdales 3 et 3' est déterminée en vue d'une adaptation optimale des lignes à retard pour une fréquence de 915 MHz ou de 434 MHz à l'émission et simultanément sur une bande de fréquence de 2 à 4 GHz à la réception, pour la mesure radiométrique.

Le mandrin 5 tubulaire creux peut, en présence d'un câble coaxial, être partiellement emmanché sur une portion extrême dénudée 10 du conducteur filaire central 9 de la ligne de transmission coaxiale 4 et comporter une ouverture radiale 11 pour le passage et la connexion de l'extrémité 6 de la portion de conducteur hélicoïdale 3, formant première ligne à retard rayonnante, sur ladite portion dénudée 10 dudit conducteur filaire central 9.

La portion de conducteur hélicoïdale 3', formant seconde ligne à retard rayonnante, est avantageusement enroulée sur la gaine extérieure 5' de la ligne de transmission coaxiale 4 et est connectée sur une portion extrême dénudée 10' du conducteur tubulaire externe 9' de cette dernière.

Afin d'assurer une isolation de l'antenne 1 par rapport aux tissus ou aux liquides environnants, les lignes à retard rayonnantes 3, 3' sont recouvertes d'une gaine 12 en un matériau diélectrique thermorétractable, par exemple en un matériau du type PTFE (polytétrafluoroéthylène) connu sous la désignation TEFLON (marque déposée).

Ladite gaine 12 thermorétractable pourra recouvrir de manière étanche la totalité de l'extrémité libre de la ligne d'alimentation 4 portant les lignes à retard 3, 3', un élément 13 en forme de bouchon, sur lequel la gaine 12 est appliquée par rétraction, après chauffage, obturant ladite gaine 12 en avant de ladite extrémité libre de ladite ligne d'alimentation 4.

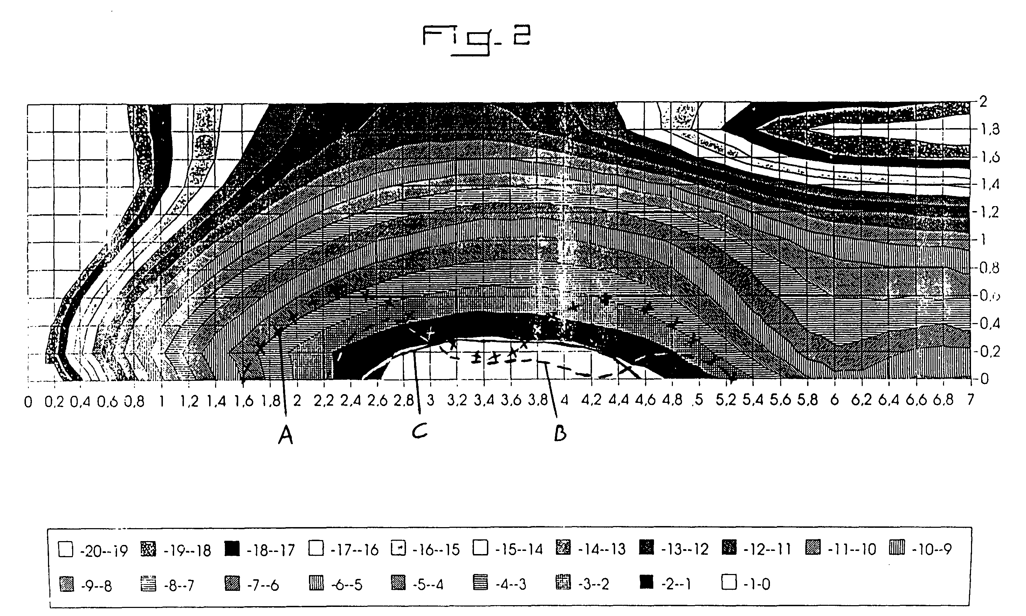

Le diagramme de la figure 2 montre, au moyen de strates isothermes (foncé: température élevée; clair: température basse - voir légende), la configuration du dépôt d'énergie obtenue au moyen d'une antenne 1 telle que celle représentée à la figure 1 et adaptée spécifiquement au traitement de la prostate au moyen d'une antenne 1 ou sonde intra-urétrale (largeur des portions de conducteur 3, 3': 2 mm, pas: 2,5 mm, diamètre d'enroulement: 2,5 mm, structure des portions 3, 3': 6 spires sur 27 mm).

Comme on le voit aisément sur cette figure, les lignes à retard 3, 3' rayonnantes répartissent l'énergie fournie par la ligne d'alimentation 4, de façon quasi homogène et symétrique, autour d'un segment de longueur définie de ladite antenne 1, dans un volume symétrique en forme de manchon nettement délimité, pour lequel l'axe longitudinal 8 de la partie rayonnante 2 constitue l'axe de symétrie.

De plus, aux limites dudit volume, une décroissance très forte de la quantité d'énergie déposée par ladite antenne, et donc une irradiation très faible en dehors dudit volume, est également nettement visible sur ledit diagramme.

En outre, la structure d'antenne décrite ci-dessus entraîne une superposition des diagrammes de rayonnement d'émission et de réception, de telle manière qu'ils sont confondus ou au moins concentriques. Ainsi, la figure 2 montre, à titre d'exemples, les courbes de rayonnement à 3 dB (50%) en réception radiométrique à 2 GHz(A), à 3 GHz(B) et à 4 GHz(C).

L'étude de cette figure permet de constater que, du fait de la quasi-superposition des diagrammes de rayonnement, à l'émission et à la réception, le volume dans lequel la température est mesurée par voie radiométrique correspond approximativement au volume chauffé à l'émission.

En vue d'adapter le diagramme de rayonnement à la configuration, la forme et la constitution des tissus ou de l'organe à traiter, il est possible de fixer à une valeur prédéterminée le nombre de spires, la largeur ou section des portions de conducteur 3, 3', le diamètre du mandrin 5, le pas d'enroulement et l'épaisseur des matériaux diélectriques (mandrin 5, gaine 12) ou éventuellement faire varier progressivement ces paramètres pour une même antenne 1.

De plus, il est également possible d'influencer le diagramme de rayonnement par une structure asymétrique des deux portions de conducteur hélicoïdales 3 et 3' ou encore par une inversion du sens d'enroulement sur un segment donné de l'une ou des deux portions de conducteur hélicoïdales 3 et 3'.

Enfin ladite antenne 1 peut éventuellement comporter plusieurs (plus de deux) lignes à retard rayonnantes 3, 3' alimentées, de manière séparée, par des signaux déphasés entre eux, ledit déphasage pouvant, le cas échéant, être soumis à des variations contrôlées, en vue de réaliser des compositions de champs complexes.

L'invention a également pour objet une sonde pour le chauffage de tissus par micro-ondes et pour la mesure de température par radiométrie micro-onde, ladite sonde comprenant au moins une antenne 1 telle que décrite ci-dessus.

Selon une première variante de réalisation de l'invention, non représentée aux dessins annexés, la sonde peut être essentiellement constituée par une unique antenne, le cas échéant disposée dans un tube ou un cathéter parcouru par un flux de fluide, notamment de liquide, de thermostatisation.

Conformément à une seconde variante de réalisation de l'invention, représentée par exemple à la figure 4 des dessins annexés, la sonde peut avantageusement comporter au moins deux antennes 1 montées dans une structure support 14, 15, 15' permettant une manipulation simultanée et un positionnement in situ exact desdites antennes 1.

La structure support pourra consister essentiellement, d'une part, en un corps tubulaire 14 autour duquel sont disposées au moins les parties rayonnantes 2 de plusieurs antennes 1 et, d'autre part, en des moyens 15, 15' de maintien et de solidarisation des antennes 1 autour dudit corps tubulaire 14.

Selon un mode de réalisation préféré de l'invention, la sonde précitée, par exemple du type vaginale ou rectale, peut comprendre une portion de tube métallique creux 14 en un matériau isolant, pouvant comporter un corps central réflecteur métallique 14' et être pourvue de deux bagues de solidarisation 15 et 15' assurant le maintien des antennes 1, au moins au niveau des extrémités 6' et 7' de leurs parties rayonnantes 2, un conduit d'amenée 16, relié à ladite portion de tube 14 et au moins un conduit d'évacuation 17, traversant la bague 15' non distale, assurant une circulation de liquide de thermostatisation autour des parties rayonnantes 2 des antennes 1, de manière à créer un manchon de liquide de thermostatisation entre lesdites bagues 15 et 15'.

Le réflecteur métallique 14' creux peut être de section cylindrique, carré ou triangulaire, afin de réflechir l'énergie rayonnée par les antennes 1 vers le milieu à traiter.

En outre une chemise extérieure pourra recouvrir l'ensemble de la sonde, afin notamment de canaliser le fluide de thermostatisation.

La sonde décrite ci-dessus constitue généralement une sonde endocavitaire pouvant être mise en oeuvre pour des applications en urologie, en gynécologie ou en cancérologie, les antennes 1 qui en font partie pourront être disposées de manière régulière ou non autour de la portion de tube 14 et être alimentées par des signaux en phase ou déphasées entre eux, ce en fonction notamment de la forme souhaitée du lobe de rayonnement.

Ces antennes 1 peuvent être alimentées:
1) en phase dans le cas d'une répartition de champ homogène;
2) avec un déphasage fixe dans le cas d'une focalisation de l'énergie dans une direction déterminée;
3) avec un déphasage variable dans le temps dans le cas d'une composition de champ balayant.

Bien entendu, l'invention n'est pas limitée au mode de réalisation décrit et représenté aux dessins annexés. Des modifications restent possibles, notamment du point de vue de la constitution des divers éléments, ou par substitution d'équivalents techniques, sans sortir pour autant du domaine de protection de l'invention.

## Revendications

1. Antenne pour le chauffage de tissus par micro-ondes, destinée à être insérée dans une cavité ou un conduit, naturel ou réalisé pour les besoins d'un traitement, d'un corps vivant, notamment humain, antenne constituée par deux lignes à retard rayonnantes (3,3') à structure hélicoïdale, présentant des sens d'enroulement opposés en partant de leurs extrémités (6,7) adjacentes respectives, montées, de manière contiguë , lesdites portions de conducteur hélicoïdales (3,3') étant connectées, au niveau de leurs extrémités adjacentes (6, 7), à deux conducteurs (9 et 9'), leurs extrémités mutuellement opposées étant non connectées, de manière à constituer un dipôle rayonnant, antenne **caractérisée en ce que** les deux lignes à retard rayonnantes (3, 3') sont montées sur des supports (5,5') en des matériaux diélectriques dont les axes longitudinaux (8) sont confondus, et se présentent sous la forme de deux portions distinctes de ruban de matériau conducteur alimenté en son centre et sont disposées au niveau de l'extrémité libre d'une ligne d'alimentation (4) en étant connectées aux deux conducteurs (9,9') de cette dernière, formant ainsi un dipôle alimenté en son centre, ladite antenne (1) autorisant également, en alternance avec le chauffage des tissus, une réception des signaux et bruits thermiques des tissus chauffés dans une bande de fréquence de 2 GHz à 4 GHz, en vue d'une détermination de la température par mesure radiométrique.

2. Antenne selon la revendication 1, **caractérisée en ce que** les deux portions de conducteur hélicoïdales (3 et 3') présentent des longueurs et des structures identiques.

3. Antenne selon l'une quelconque des revendications 1 et 2, **caractérisée en ce que** chaque portion de conducteur hélicoïdale (3, 3') est constituée par un ruban en un matériau conducteur dont la largeur, le pas d'enroulement, le diamètre d'enroulement et le nombre de spires formées sont déterminés en fonction de la forme du diagramme de rayonnement et de l'adaptation de l'antenne à la fréquence désirée.

4. Antenne selon la revendication 3, **caractérisée en ce que** la longueur des portions de conducteur hélicoïdales (3 et 3') est déterminée en vue d'une adaptation optimale des lignes à retard pour une fréquence de 915 MHz ou de 434 MHz à l'émission et simultanément sur une bande de fréquence de 2 à 4 GHz à la réception.

5. Antenne selon l'une quelconque des revendications 1 à 4, **caractérisé en ce que** les diagrammes de rayonnement à l'émission et à la réception sont confondus, ou au moins concentriques.

6. Antenne selon l'une quelconque des revendications 1 à 5, **caractérisée en ce que** la ligne d'alimentation (4) consiste en une ligne de transmission à structure coaxiale.

7. Antenne selon l'une quelconque des revendications 1 à 6, **caractérisée en ce que** la portion de conducteur (3) formant première ligne à retard rayonnante est enroulée sur un support d'enroulement (5) en forme de mandrin tubulaire creux et à section variable.

8. Antenne selon les revendications 6 et 7, **caractérisée en ce que** le mandrin tubulaire creux (5) est partiellement emmanché sur une portion extrême dénudée (10) du conducteur filaire central (9) de la ligne de transmission coaxiale (4) et comporte une ouverture radiale (11) pour le passage et la connexion de l'extrémité (6) de la portion de conducteur hélicoïdale (3), formant première ligne à retard rayonnante, sur ladite portion dénudée (10) dudit conducteur filaire central (9).

9. Antenne selon l'une quelconque des revendications 6 à 8, **caractérisée en ce que** la portion de conducteur hélicoïdale (3') formant une seconde ligne à retard rayonnante est enroulée sur la gaine extérieure (5') de la ligne de transmission coaxiale (4) et est connectée sur une portion extrême dénudée (10') du conducteur tubulaire externe (9') de cette dernière.

10. Antenne selon l'une quelconque des revendications 1 à 9, **caractérisée en ce que** les lignes à retard rayonnantes (3, 3') sont recouvertes d'une gaine (12) en un matériau diélectrique thermorétractable.

11. Antenne selon la revendication 10, **caractérisée en ce que** la gaine (12) thermorétractable recouvre de manière étanche la totalité de l'extrémité libre de la ligne d'alimentation (4) portant les lignes à retard (3, 3'), un élément (13) en forme de bouchon, sur lequel la gaine (12) est appliquée par rétraction, après chauffage, obturant ladite gaine (12) en avant de ladite extrémité libre de ladite ligne d'alimentation (4).

12. Antenne selon l'une quelconque des revendications 1 à 11, **caractérisée en ce que** les lignes à retard rayonnantes (3, 3') sont alimentées, de manière séparée, par des signaux déphasés entre eux, ledit déphasage pouvant être soumis à des variations contrôlées.

13. Sonde pour le chauffage de tissus par micro-ondes et pour la mesure de température par radiométrie micro-onde, **caractérisée en ce qu'**elle comprend au moins une antenne (1) selon l'une quelconque des revendications 1 à 12.

14. Sonde selon la revendication 13, **caractérisée en ce qu'**elle est constituée par une unique antenne disposée dans un tube ou un cathéter parcouru par un flux de fluide, notamment de liquide, de thermostatisation, de manière à créer un manchon continu de liquide de thermostatisation entourant directement les parties rayonnantes (2) au moins de ladite antenne.

15. Sonde selon la revendication 13 **caractérisée en ce qu'**elle comporte au moins deux antennes (1) montées dans une structure support (14, 15, 15') permettant une manipulation simultanée et un positionnement in situ exact desdites antennes (1).

16. Sonde selon la revendication 15, **caractérisée en ce que** la structure support consiste d'une part, en un corps tubulaire (14) autour duquel sont disposées au moins les parties rayonnantes (2) de plusieurs antennes (1) et, d'autre part, en des moyens (15, 15') de maintien et de solidarisation des antennes (1) autour dudit corps tubulaire (14).

17. Sonde selon la revendication 16 **caractérisée en ce qu'**elle comprend une portion de tube creux (14) en un matériau isolant, comportant un corps central réflecteur métallique (14') et pourvue de deux bagues de solidarisation (15 et 15') assurant le maintien des antennes (1), au moins au niveau des extrémités (6' et 7') de leurs parties rayonnantes (2), un conduit d'amenée (16), relié à ladite portion de tube (14), et au moins un conduit d'évacuation (17), traversant la bague (15') non distale, assurant une circulation de liquide de thermostatisation autour des parties rayonnantes (2) des antennes (1), de manière à créer un manchon continu de liquide de thermostatisation entre lesdites bagues (15 et 15') entourant directement lesdites parties rayonnantes (2).

18. Sonde selon l'une quelconque des revendications 13 à 17, **caractérisée en ce qu'**elle constitue une sonde endocavitaire pouvant être mise en oeuvre pour des applications en urologie, en gynécologie ou en cancérologie, et comportant des antennes (1) alimentées par des signaux en phase ou déphasés entre eux.

## Patentansprüche

1. Antenne zum Erhitzen von Gewebe durch Mikrowellen, die zum Einsetzen in einer natürlichen oder zu Behandlungszwecken hergestellten Höhle oder Kanal eines lebendigen Körpers, insbesondere eines menschlichen, bestimmt ist, wobei die Antenne gebildet ist durch zwei strahlende Laufzeitleitungen (3, 3') mit spiralförmiger Struktur, die ausgehend von ihren jeweils benachbarten Enden (6, 7) , entgegengesetzte Wickelsinne aufweisen, die aneinander angrenzend befestigt sind, wobei die spiralförmigen Leiterabschnitte (3, 3') auf Höhe ihrer benachbarten Enden (6, 7) mit zwei Leitern (9 und 9') verbunden sind, wobei deren gegenseitig entgegengesetzten Enden nicht verbunden sind, derart dass ein strahlender Dipol gebildet wird, wobei die Antenne **dadurch gekennzeichnet ist, dass** die beiden strahlenden Laufzeitleitungen (3, 3') auf Trägern (5, 5') aus dielektrischen Materialien, deren Längsachsen (8) zusammenlaufen, befestigt sind und sich in Form von zwei unterschiedlichen Bandabschnitten aus einem in seinem Zentrum gespeisten Leitermaterial darstellen und auf Höhe des freien Endes einer Speiseleitung (4) angeordnet sind, indem sie mit den zwei Leitern (9, 9') von letzterer verbunden sind und so einen in seinem Zentrum gespeisten Dipol bilden, wobei die Antenne (1) auch, alternativ zum Erhitzen des Gewebes, einen Empfang von thermischen Signalen und Rauschen der erhitzten Gewebe in einem Frequenzband von 2 GHz bis 4 GHz, zur Bestimmung der Temperatur durch radiometrische Messung, ermöglicht.

2. Antenne nach Anspruch 1, **dadurch gekennzeichnet, dass** die beiden spiralförmigen Leiterabschnitte (3 und 3') identische Längen und Strukturen aufweisen.

3. Antenne nach einem der Ansprüche 1 und 2, **dadurch gekennzeichnet, dass** jeder spiralförmige Leiterabschnitt (3, 3') durch ein Band aus einem leitfähigen Material gebildet ist, dessen Breite, Wickelschritt, Wickeldurchmesser und Anzahl von gebildeten Windungen bestimmt sind in Abhängigkeit der Form des Strahlungsdiagramms und der Anpassung der Antenne an die gewünschte Frequenz.

4. Antenne nach Anspruch 3, **dadurch gekennzeichnet, dass** die Länge der spiralförmigen Leiterabschnitte (3 und 3') bestimmt ist im Hinblick auf eine optimale Anpassung der Laufzeitleitungen für eine Frequenz von 915 MHz oder 434 MHz bei der Emission und gleichzeitig auf ein Frequenzband von 2 bis 4 GHz beim Empfang.

5. Antenne nach einem der Ansprüche 1 bis 4, **dadurch gekennzeichnet, dass** die Strahlungsdiagramme bei der Emission und beim Empfang vermischt sind oder wenigstens konzentrisch verlaufen.

6. Antenne nach einem der Ansprüche 1 bis 5, **dadurch gekennzeichnet, dass** die Speiseleitung (4) aus einer Transmissionsleitung mit koaxialer Struktur besteht.

7. Antenne nach einem der Ansprüche 1 bis 6, **dadurch gekennzeichnet, dass** der Leiterabschnitt (3), der die erste strahlende Laufzeitleitung bildet auf einen Aufwickelträger (5) in Form eines hohlen, rohrförmigen Dorns mit variablem Durchmesser aufgewickelt ist.

8. Antenne nach den Ansprüchen 6 und 7, **dadurch gekennzeichnet, dass** der hohle, rohrförmige Dorn (5) teilweise aufgesteckt ist auf einen abgemantelten End- Abschnitt (10) des zentralen drahtförmigen Leiters (9) der koaxialen Übertragungsleitung (4) und eine radiale Öffnung (11) aufweist zum Durchtritt und Verbinden des Endes (6) des spiralförmigen Leiterabschnitts (3), der die erste strahlende Laufzeitleitung bildet, an dem abgemantelten Abschnitt (10) des zentralen, drahtförmigen Leiters (9).

9. Antenne nach einem der Ansprüche 6 bis 8, **dadurch gekennzeichnet, dass** der spiralförmige Leiterabschnitt (3'), der eine zweite strahlende Laufzeitleitung bildet, auf die äußere Hülle (5') der koaxialen Übertragungsleitung (4) aufgewickelt ist und an einem abgemantelten EndAbschnitt (10') des äußeren rohrförmigen Leiters (9') von letzterer verbunden ist.

10. Antenne nach einem der Ansprüche 1 bis 9, **dadurch gekennzeichnet, dass** die strahlenden Laufzeitleitungen (3, 3') mit einer Hülle (12) aus einem dielektrischen Material mit Wärmeschrumpfung bedeckt sind.

11. Antenne nach Anspruch 10, **dadurch gekennzeichnet, dass** die wärmeschrumpfbare Hülle (12) das gesamte freie Ende der Speiseitung (4), die die Laufzeitleitungen (3,3') trägt, ein Element (13) in Form eines Stopfens dicht bedeckt, auf das die Hülle (12) durch Schrumpfen (Retraktion), nach Erhitzen, angebracht wird, wodurch die Hülle (12) vor dem freien Ende der Speiseleitung (4) verschlossen wird.

12. Antenne nach einem der Ansprüche 1 bis 11, **dadurch gekennzeichnet, dass** die strahlenden Laufzeitleitungen (3, 3') getrennt durch untereinander phasenverschobene Signale gespeist werden, wobei die Phasenverschiebung kontrollierten Schwankungen unterworfen sein kann.

13. Sonde zum Erhitzen von Gewebe durch Mikrowellen und zum Messen der Temperatur durch Mikrowellen-Radiometrie, **dadurch gekennzeichnet, dass** sie wenigstens eine Antenne (1) nach einem der Ansprüche 1 bis 12 aufweist.

14. Sonde nach Anspruch 13, **dadurch gekennzeichnet, dass** sie gebildet ist durch eine einzige Antenne, die in einem von einem Fluidstrom, insbesondere Flüssigkeit durchlaufenen Rohr oder Katheter zur Temperaturregelung angeordnet ist, derart dass ein kontinuierlicher Mantel von Flüssigkeit zur Temperaturregelung geschaffen wird, der direkt die strahlenden Teile (2) wenigstens der Antenne umgibt.

15. Sonde nach Anspruch 13, **dadurch gekennzeichnet, dass** sie wenigstens zwei Antennen (1) aufweist, die in einer Trägerstruktur (14, 15, 15') befestigt sind, die die gleichzeitige Handhabung und ein exaktes Positionieren in situ der Antennen (1) ermöglicht.

16. Sonde nach Anspruch 15, **dadurch gekennzeichnet, dass** die Trägerstruktur einerseits aus einem rohrförmigen Körper (14), um den herum wenigstens die strahlenden Teile (2) von mehreren Antennen (1) angeordnet sind, und andererseits aus Mitteln (15, 15') zum Halten und Befestigen der Antennen (1) um diesen rohrförmigen Körper (14) herum besteht.

17. Sonde nach Anspruch 16, **dadurch gekennzeichnet, dass** sie einen hohlen Rohrabschnitt (14) aus einem isolierenden Material, der einen metallischen, reflektierenden, zentralen Körper (14') aufweist und mit zwei Befestigungsringen (15 und 15') versehen ist, die das Halten der Antennen (1) wenigstens auf der Höhe der Enden (6' und 7') ihrer strahlenden Teile (2) gewährleisten, eine Zulaufleitung (16), die mit dem Rohrabschnitt (14) verbunden ist, und wenigstens eine Abführleitung (17) aufweist, die den nicht distalen Ring (15') durchquert, wodurch eine Zirkulation von Flüssigkeit zur Temperaturregelung um die strahlenden Teile (2) der Antennen (1) gewährleistet wird, derart, dass ein kontinuierlicher Mantel von Flüssigkeit zur Temperaturregelung zwischen den Ringen (15 und 15'), der direkt die strahlenden Teile (2) umgibt, geschaffen wird.

18. Sonde nach einem der Ansprüche 13 bis 17, **dadurch gekennzeichnet, dass** sie eine endokavitäre Sonde bildet, die bei Anwendungen in der Urologie, Gynäkologie oder in der Cancerologie eingesetzt werden kann und Antennen (1) aufweist, die durch Signale in Phase oder untereinander phasenverschoben gespeist werden.

## Claims

1. Antenna for microwave heating of tissues, which is designed to be inserted in a cavity or a duct which is natural or is created for the needs of a treatment of a living body, and in particular a human body, which antenna consists of two radiating retard lines (3, 3') with a helical structure, which have opposite directions of winding starting from their respective adjacent ends (6, 7), and are fitted in a contiguous manner, the said portions of helical conductor (3, 3') being connected at the level of their adjacent ends (6, 7) to two conductors (9 and 9'), their mutually opposite ends not being connected, such as to constitute a radiating dipole, which antenna is **characterised in that** the two radiating retard lines (3, 3') are fitted on supports (5, 5') in dielectric devices, the longitudinal axes (8) of which are combined, and are in the form of two distinct portions of strips of conductive material which is supplied at its centre, and are disposed at the level of the free end of a supply line (4) whilst being connected to the two conductors (9, 9') of the latter, thus forming a dipole which is supplied at its centre, the said antenna (1) also permitting, in alternance with the heating of the tissues, receipt of signals and thermal noise of the tissues heated, in a frequency band of 2 GHz to 4 GHz, for the purpose of determination of the temperature by radiometric measurement.

2. Antenna according to claim 1, **characterised in that** the two portions of helical conductor (3 and 3') have identical lengths and structures.

3. Antenna according to either of claims 1 and 2, **characterised in that** each portion of helical conductor (3, 3') consists of a strip made of a conductive material, the width, winding pitch, winding diameter and number of turns formed of which are determined according to the form of the radiation diagram and of adaptation of the antenna to the required frequency.

4. Antenna according to claim 3, **characterised in that** the length of the portions of helical conductor (3 and 3') is determined for the purpose of optimal adaptation of the retard lines for a frequency of 915 MHz or 434 MHz at emission and similarly on a frequency band of 2 to 4 GHz at reception.

5. Antenna according to any one of claims 1 to 4, **characterised in that** the radiation diagrams at emission and reception are combined, or at least concentric.

6. Antenna according to any one of claims 1 to 5, **characterised in that** the supply line (4) consists of a transmission line with a coaxial structure.

7. Antenna according to any one of claims 1 to 6, **characterised in that** the portion of conductor (3) which forms the first radiating retard line is wound on a winding support (5) in the form of a hollow tubular mandrel with a variable cross-section.

8. Antenna according to claim 6 and claim 7, **characterised in that** the hollow tubular mandrel (5) is partially fitted onto a bare end portion (10) of the central filar conductor (9) of the coaxial transmission line (4) and comprises a radial aperture (11) for the passage and connection of the end (6) of the helical conductor portion (3), forming a first radiating retard line, on the said bare portion (10) of the said central filar conductor (9).

9. Antenna according to any one of claims 6 to 8, **characterised in that** the portion of the helical conductor (3') which forms a second radiating retard line is wound on the external sheath (5') of the coaxial transmission line (4) and is connected to a bare end portion (10') of the external tubular conductor (9') of the latter.

10. Antenna according to any one of claims 1 to 9, **characterised in that** the radiating retard lines (3, 3') are covered with a sheath (12) made of a heat-shrinkable dielectric material.

11. Antenna according to claim 10, **characterised in that** the heat-shrinkable sheath (12) covers in a sealed manner all of the free end of the supply line (4) which supports the retard lines (3, 3'), an element (13) in the form of a stopper, onto which the sheath (12) is applied by being shrunk after being heated, thus sealing the said sheath (12) on the front of the said free end of the said supply line (4).

12. Antenna according to any one of claims 1 to 11, **characterised in that** the radiating retard lines (3, 3') are supplied in a separate manner by signals which are out of phase with one another, the said phase displacement being able to be subjected to controlled variations.

13. Probe for microwave heating of tissues and for temperature measurement by microwave radiometry, **characterised in that** it comprises at least one antenna (1) according to any one of claims 1 to 12.

14. Probe according to claim 13, **characterised in that** it consists of a single antenna disposed in a tube or a catheter through which a flow of thermostatting fluid, and in particular liquid, passes, such as to create a continuous sleeve of thermostatting liquid which surrounds directly the radiating parts (2) at least of the said antenna.

15. Probe according to claim 13, **characterised in that** it comprises at least two antennae (1) which are fitted in a support structure (14, 15, 15') which permits simultaneous handling and accurate positioning in situ of the said antennae (1).

16. Probe according to claim 15, **characterised in that** the support structure consists firstly of a tubular body (14) around which there are disposed at least the radiating parts (2) of a plurality of antennae (1), and secondly of means (15, 15') for retention and integration of the antennae (1) around the said tubular body (14).

17. Probe according to claim 16, **characterised in that** it comprises a portion of hollow tube (14) which is made of an insulating material, comprising a metal reflective central body (14') and being provided with two integration rings (15 and 15') which assure retention of the antennae (1), at least at the level of the ends (6' and 7') of their radiating part (2), an intake duct (16) which is connected to the said portion of tube (14), and at least one discharge duct (17) which passes through the non-distal ring (15'), thus assuring circulation of the thermostatting liquid around radiating parts (2) of the antennae (1), such as to create a continuous sleeve of thermostatting liquid between the said rings (15 and 15') which surround the said radiating parts (2) directly.

18. Probe according to any one of claims 13 to 17, **characterised in that** it constitutes an endocavity probe which can be implemented for applications in urology, gynaecology, or cancerology, and comprising antennae (1) which are supplied by signals which are in phase or out of phase with one another.
